(19) **Europäisches Patentamt European Patent Office Office européen des brevets**

(11) **EP 3 860 682 B1**

(12) **EUROPEAN PATENT SPECIFICATION**

(45) Date of publication and mention of the grant of the patent:
**29.10.2025 Bulletin 2025/44**

(21) Application number: **19783822.0**

(22) Date of filing: **24.09.2019**

(51) International Patent Classification (IPC):
*A61M 5/168* (2006.01)    *A61M 5/36* (2006.01)
*A61M 5/142* (2006.01)

(52) Cooperative Patent Classification (CPC):
**A61M 5/16854; A61M 5/365;** A61M 5/14244;
A61M 2005/16863; A61M 2205/3306;
A61M 2205/332

(86) International application number:
**PCT/US2019/052578**

(87) International publication number:
**WO 2020/072234 (09.04.2020 Gazette 2020/15)**

(54) **DETECTION ASSEMBLIES FOR INFUSION PUMPS**

DETEKTIONSANORDNUNGEN FÜR INFUSIONSPUMPEN

ASSEMBLAGES DE DETECTION POUR POMPES A PERFUSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.10.2018 US 201862740585 P**

(43) Date of publication of application:
**11.08.2021 Bulletin 2021/32**

(73) Proprietor: **Takeda Pharmaceutical Company
Limited
Osaka-shi,
Osaka (JP)**

(72) Inventors:
• **ARGERSINGER, Andrew, Scott**
**West Bristol, WI 53104 (US)**
• **BYERS, Brent, Whitfield**
**Plano, TX 75093 (US)**
• **STUBBS, Jason, Paul**
**Southlake, TX 76092 (US)**

(74) Representative: **Carpmaels & Ransford LLP
One Southampton Row
London WC1B 5HA (GB)**

(56) References cited:
WO-A2-99/64091    US-A- 5 554 115
US-A- 5 661 245    US-A1- 2003 200 812

EP 3 860 682 B1

**Description**

FIELD

Disclosed embodiments are related to detection systems for infusion pumps and related medicinal fluid delivery systems.

BACKGROUND

Medicinal fluids are administered to patients through a variety of methods. These conventional methods typically include injection by a syringe, ingestion, or delivery by an infusion pump and needle. In the case of administration by an infusion pump, controlled volumes of medicinal fluids may be delivered to the patient at pre-programmed rates or automated intervals.

The delivery of medicinal fluids by an infusion pump has, historically, been performed and monitored by an experienced health care provider who is responsible for operating the infusion pump to administer medicinal fluid as well as any maintenance or cleaning the infusion pump might require. However, constant monitoring of proper fluid delivery by a health care provider is not always possible. For example, health care providers often need to look after multiple patients at a time. As another example, smaller outpatient infusion pumps have become widely available in recent years, and patients themselves and/or family members, rather than health care providers, may operate such pumps themselves at home.

US 5 554 115 A discloses a pressure sensor that responds to stress introduced into a pair of cantilevered beams, to sense pressures at two measurement points in a cassette.

WO 99/64091 A2 discloses a system and a method for detecting the presence of an occlusion in an intravenous (IV) line supplying a medicinal liquid to a patient.

US 5 661 245 A discloses a force sensor assembly which incorporates an integrated plunger to provide an economical assembly which can be calibrated and mounted as a single unit.

US 2003/200812 A1 discloses an invention which lessens the amount of air entering between mating membranes of a pressure sensor.

SUMMARY

The invention comprises an infusion pump as defined in claim 1 and a method of monitoring an infusion pump according to claim 9. Further embodiments of the invention are defined in the dependent claims. In some embodiments, systems and methods for monitoring an infusion pump for occlusions or bubbles (e.g., conditions) in the delivery stream are disclosed. In some embodiments, systems and methods of detecting an upstream or downstream occlusion with the occlusion detector or bubble detector are described. In some embodiments, systems and methods for estimating a delivered volume of medicinal fluid and using said estimated volume to differentiate between a detected occlusion and the end of medicinal fluid delivery are disclosed.

In some embodiments, the occlusion detector comprises a membrane that deforms in response to fluid pressure generated by medicinal fluid delivery through the pump, a piston which the membrane exerts axial force on when deforming, and a force sensor that indirectly measures fluid pressure within the pump. In some embodiments, the bubble detector includes a light sensor and light emitter that shines light through the medicinal fluid flow path of the infusion pump. The light refracts differently as it passes through the flow path depending on how much air or fluid is within the fluid flow path. The light is detected by the light sensor, which relays an output signal to a controller of the infusion pump.

In some embodiments, an infusion pump comprises a pump engine having an inlet and an outlet, the pump engine being configured to drive fluid in a direction from the inlet to the outlet along a first fluid flow path; a membrane that is non-porous; a piston; a second fluid flow path in fluid communication with the first fluid flow path, wherein the second fluid flow path terminates at the membrane and piston; a force sensor; and a controller in communication with the force sensor. In some embodiments, the force sensor is configured to send a signal to the controller in response to deformation of the membrane, reflecting fluid pressure experienced in the fluid flow path. A first fluid pressure in the first fluid flow path gives rise to a second fluid pressure in the second fluid flow path. The membrane is exposed to, and is configured to, deform in response to the second fluid pressure in the second fluid flow path, and the piston is configured to move and to propagate an axial force to the force sensor in response to deformation of the membrane.

In some embodiments, a method of monitoring an infusion pump comprises determining a threshold value based on a flow rate of a first fluid flow when a pump engine is activated to deliver fluid from an inlet to an outlet across a first fluid flow path such that fluid also enters a second fluid flow path deviating from the first fluid flow path; deforming a non-porous membrane at an end of the second fluid flow path to apply an axial force to a piston operatively connected to a force sensor which delivers a first reading; detecting a second reading when an occlusion at least partially obstructs the first fluid flow path, increasing fluid in the second fluid flow path, increasing axial force on the piston through an increase in pressure further deforming the membrane; and alerting a user if the second reading surpasses the threshold value.

In some embodiments, a method of monitoring an infusion pump comprises: delivering fluid from an inlet to an outlet across a first fluid flow path using a rotary pump with a known rotation rate and volume delivered per rotation; sensing a first pressure value with a force sensor, wherein fluid from the first fluid flow path flows into a second fluid flow path in fluid communication with the first fluid flow path, deforming a non-porous membrane in the second fluid flow path, applying axial force to a piston operatively connected to the force sensor; sensing a second pressure value different from the first pressure value; determining a delivered volume based on a number of rotations of the rotary pump; and triggering an alert if delivered volume is below an expected volume threshold.

In some embodiments, a method of monitoring an infusion pump comprises delivering fluid from an inlet to an outlet across a first fluid flow path using a rotary pump with a known volume delivered per rotation and rotation rate; monitoring the first fluid flow path with an optical sensor; detecting if air is present in the first fluid flow path; determining a delivered volume based on a number of rotations of the rotary pump; and triggering a user alert if delivered volume is below an expected volume threshold.

It should be appreciated that the foregoing concepts, and additional concepts discussed below, may be arranged in any suitable combination, as the present disclosure is not limited in this respect. Further, other advantages and novel features of the present disclosure will become apparent from the following detailed description of various non-limiting embodiments when considered in conjunction with the accompanying figures.

BRIEF DESCRIPTION OF DRAWINGS

The accompanying drawings are not intended to be drawn to scale. In the drawings, each identical or nearly identical component that is illustrated in various figures may be represented by a like numeral. For purposes of clarity, not every component may be labeled in every drawing. In the drawings:

FIG. 1 is a perspective view of an embodiment of an infusion pump;
FIG. 2 is a perspective view of the infusion pump of FIG. 1 with a compartment lid removed;
FIG. 3 is a perspective view of an embodiment of a pump engine with an occlusion detector according to a first embodiment and a bubble detector according to one embodiment;
FIG. 4 is a front view of the pump engine including the occlusion detector and the bubble detector according to the embodiment of Fig. 3;
FIG. 5 is a close up front view of the occlusion detector according to one embodiment;
FIG. 6 is a partial cross-sectional view of the infusion pump with the occlusion detector according to one embodiment;
FIG. 7 is an exploded perspective view of part of the occlusion detector according to one embodiment;
FIG. 8 is a close up front view of the pump engine with the bubble detector according to one embodiment;
FIG. 9 is a partial cross-sectional view of the pump engine according to one embodiment;
FIG. 10 is a block diagram illustrating the operation of the bubble detector according to one embodiment;
FIG. 11 is a table containing values used to determine a pressure threshold for downstream occlusion detection during the delivery of a medicinal fluid according to one example; and
FIG. 12 is a line graph showing example downstream occlusion thresholds by flow rate according to one example.

DETAILED DESCRIPTION

When infusing medicinal fluids using an infusion pump, for various reasons, it is possible for the medicinal lines to become occluded. For example, excess movement or activity around or by the infusing patient could lead to disconnections or kinking of the infusion set, users can make errors when clamping delivery lines, and/or blood clotting around a delivery site could all cause occlusions. Occlusion of the delivery lines inhibits medication from reaching the patient, while extended periods of occlusion could damage various parts of the infusion pump.

For various reasons, air could become trapped within the medicinal fluid to be delivered to a patient. Depending on the infusion location, air bubbles in the delivered fluid can cause discomfort and/or interfere with treatment.

The inventors have recognized the need for providing infusion pumps with detectors for detection of occlusions and/or bubbles (e.g., conditions).

The inventors have further contemplated that a bubble detector in some embodiments could be used to assist in monitoring other aspects associated with delivery of the medicinal fluid. In some embodiments, the bubble detector can be used to determine that delivery has been completed. For instance, the detection of an extended air bubble could correspond to having detected the trailing edge of the medicinal fluid at the end of delivery.

In some embodiments, the bubble detector may draw from other information monitored by the infusion pump to determine if detection of air signifies an occlusion or the end of medicinal fluid delivery. In some embodiments, the infusion pump monitors or estimates how much medicinal fluid has been delivered, e.g., using flow sensors while tracking elapsed time, monitoring the number of turns of the infusion pump motor and/or a rotor of the pump engine, etc. Using this information, the infusion pump can react differently in response to detection of air depending on how much medicinal fluid has been estimated to have been delivered. For instance, if the other sensors in the infusion pump indicate a high likelihood that all of the medication has been delivered at the same time that the bubble detector detects air in the line, no air or occlusion alarm is triggered. Instead, the detection of air may serve to confirm that the entirety of the medicinal fluid dose has been delivered and the infusion pump alerts the user to the end of infusion. However, if the bubble detector detects air in the line while other sensors in the infusion pump indicate that the total volume of expected medication has not yet been delivered, the bubble detector alerts a user or triggers an air-in-line alarm.

In some embodiments, the occlusion detector and the bubble detector are configured to work with infusion pumps that have removable pump engines (e.g., the pump engine is a disposable, one-time use pump engine that is removed and replaced after each infusion session). In these embodiments, the occlusion detector and bubble detector have removable portions that can be easily disconnected from the pump engine, and easily reattachable and connectable to the permanent portions that remain with the control unit when a new pump engine is connected.

In some embodiments of the occlusion detector, the occlusion detector is located adjacent a first fluid flow path of the medicinal fluid that connects the inlet and the outlet of the pump engine. In these embodiments, a second fluid flow path branches off from the first fluid flow path, e.g., at a substantially perpendicular angle, while maintaining fluid communication with the first fluid flow path. The second fluid flow path terminates at a membrane, which is exposed to fluid in the second fluid flow path and deforms in response to fluid pressure in the second fluid flow path. When the membrane deforms, the membrane applies an axial force to a piston, which is configured to move in response, to propagate the axial force to trigger a force sensor button. Actuating the force sensor button activates a force sensor located on a PCB, which in turn relays an output signal to the controller. A retainer produces a fluid-tight seal with the membrane to create a seal of the second fluid path. The bottom of the retainer further retains the piston, creating an upper and lower limit for the piston to translate between. The force sensor button is contained within a force sensor seal, which is an elastomeric disk which retains the button and is flexibly deformable to permit movement of the button. In some embodiments, when the force sensor button is actuated, (e.g. due a force being exerted against the force sensor button), the force sensor button is urged to translate towards a force sensor. In some embodiments, as the force exerted against the force sensor button is due to a piston that translates towards the seal and force sensor button, and presses against the force sensor button and seal. As a result of the force being exerted against the force sensor button and the seal, the seal flexes and reversibly deforms, allowing the force sensor button to translate towards and activate the force sensor. However, other embodiments with different methods of relaying pressure to the force sensor are contemplated depending on the type of force sensor utilized.

In embodiments with a removable pump engine, the force sensor and force sensor seal remain attached to the control unit. The second fluid flow path, membrane, retainer, and piston can be removed from the rest of the occlusion detector along with the pump engine. When the pump engine is attached to the control unit, the retainer is aligned with the force sensor button such that the piston comes into light contact with the force sensor button when the pump engine is fully attached. The retainer maintains a loose hold on the piston, allowing the piston to easily translate in response to deformation of the membrane, but also allowing for tolerance in the manufacturing of the connection interface between the retainer, piston, and force sensor seal holding the force sensor button.

In some embodiments, the membrane is comprised of elastomeric silicone. However, similar water resistant, flexible materials like neoprene, polyurethane, polyisoprene, nitrile, and other materials are also contemplated.

In some embodiments, the force sensor is a strain gauge load cell. However, embodiments using other types of force sensors are contemplated as long as the sensor is capable of producing output signals proportional to the force exerted on the piston by the deformed membrane. Additionally, instead of utilizing a strain gauge load cell, other embodiments could use piezoelectric force transducers, hydraulic or pneumatic load cells, linear variable differential transducers, capacitive load cells, optical strain gauges, interference-optical load cells, tuning-fork load cells, vibrating wire transducers, surface-wave resonator load cells, or any other type of force transducer in addition to, or instead of, the strain gauge load cell.

In practice, a method for detecting an occlusion using the occlusion detector according to one embodiment is as follows. During normal operations of the infusion pump, medicinal fluid is pumped from a source, into the inlet of the pump engine, and through to the outlet of the pump engine across the first fluid flow path on the way to the patient. Some of the fluid flowing through the first fluid flow path fills the second fluid flow path, producing a fluid pressure on the membrane at the end of the second fluid flow path. As the membrane deforms, the membrane produces an axial force on the piston, causing the piston to trigger the force sensor button and force sensor in turn. The force sensor sends an output signal to the controller corresponding to the pressure being experienced by the membrane. A first reading is generated during normal operation or during calibration. If an occlusion occurs upstream to the pump, fluid flow becomes partially or entirely cut off from the first fluid flow path. This reduces the fluid pressure in the second fluid flow path, reducing fluid pressure acting on the membrane, and reducing the output signal being registered by the controller. If the output signal level is below a threshold minimum value, the controller registers that an occlusion has occurred and alerts the user to a possible upstream occlusion. In some embodiments, the output signal level must be below the threshold minimum value for a pre-defined period of time and/or the signal level drops occur frequently enough as to exceed a threshold number of instances before an alarm is triggered. If an occlusion occurs downstream to the pump, the space between the pump and the occlusion rapidly fills with medicinal fluid, increasing the fluid pressure on the membrane to be higher than the level expected during normal operations. This causes the membrane to deform more, increasing the axial force on the piston, and correspondingly increasing the force experienced by the force sensor, producing an increased output signal. If the output signal level is above a threshold maximum value, the controller registers that an occlusion has occurred and alerts the user to a possible downstream occlusion. In some embodiments, the output signal level must be above the threshold maximum value for a pre-defined period of time and/or the signal level spikes occur frequently enough as to exceed a threshold number of instances before an alarm is triggered.

The methodology for determining the threshold maximum value for a downstream occlusion can differ from embodiment to embodiment, and further depends on the type and viscosity of the medicinal fluid being delivered, the diameter, length, and thickness of the delivery tubing, the diameter and length of the needle, the size, the temperature, and various other factors. When other factors are considered, the threshold value could also vary with the flow rate of the medicinal fluid. In some embodiments, the threshold is calculated by summing a worst case scenario line pressure for the flow rate (ml/hour/delivery site) based on medicinal fluid viscosity, and delivery tube geometry, with a tolerance value for safety based on the accuracy of the force sensor, and possibly an estimated body pressure of the patient depending on the delivery site. However, it should be understood that this method is just one of many possible methods of calculating a possible threshold value and the devices of the current disclosure are not limited to utilizing this specific calculation methodology.

Considering the unpredictability of fluid flow and possible jostling and movement of the infusion pump and medicinal fluid source during infusion, pressures could fluctuate above and below the thresholds even without an occlusion event occurring. Recognizing this, in some embodiments, the inventors designed the controller to record each event of the output signal exceeding, or being lower than, the thresholds, and only trigger an alarm or alert a user if there have been enough hits in a certain period. According to one embodiment of the occlusion detector, the controller samples the output threshold every second, and only triggers an alert if fourteen events of the output signal being greater than an upper threshold value or lower than a lower threshold value are detected within a certain time frame. Such a rolling time period accounts for transient pressure spikes that are not necessarily indicative of a problem, while still monitoring for actual occlusions. While a sampling time of one second and a requirement of fourteen events within a certain time period has been described, it should be understood that these parameters are merely for illustrative purposes. Actual sampling frequencies, number of event occurrences required, and time periods utilized by the occlusion detector can vary and may be chosen based on further testing.

In some embodiments, a controller may determine whether or not to provide an alert to a user based on an incremental variable which increases or decreases depending on whether an output signal exceeds or is below a threshold value. That is, during a sampling period, the controller may sample the output signal and determine if the output signal is above or at a threshold and may correspondingly increase the variable by a first value. If the output signal is below the threshold during the period, the variable may be decreased by a second value. Once the incremental variable reaches an alarm threshold, an alarm may be triggered to alert a user to a corresponding condition. In some embodiments, the first value and second value may be different, such that the controller is more responsive to conditions that may require an alarm, while still filtering aberrant or otherwise outlying signal outputs. For example, in one embodiment, the controller may sample an average pressure during a time period (e.g., a 1 second time period) and determine if that average pressure is greater than or equal to a threshold average pressure, If the average pressure is greater than or equal to the threshold pressure, an incremental variable may be increased by 2 (starting from 0). If the average pressure is less than the threshold pressure, the incremental variable may be decreased by 1 until the incremental variable is zero. According to this embodiment, an alarm for an occlusion condition may be triggered when the incremental variable reaches a value of 14 or greater (i.e., an alarm threshold). Of course, any suitable alarm threshold may be employed, and any suitable increase or decrease in value may be employed to modify the value of an incremental variable in an assigned sampling period, as the present disclosure is not so limited.

According to exemplary embodiments described herein, a controller may sample an output signal from one or more sensors for a predetermined time period (i.e., a sampling period), such that one or more operations (e.g., averaging, filtering, comparing to a threshold) may be performed on the data collected during said predetermined time period. In some embodiments, the time period may be greater than or equal to 0.125 seconds, 0.25 seconds, 0.5 seconds, 1 second, 1.5 seconds, 2 seconds, 5 seconds, and/or any other appropriate duration. Correspondingly, the time period may be less than or equal to 7.5 seconds, 5 seconds, 2 seconds, 1.5 seconds, 1 second, 0.5 seconds, 0.25 seconds, and/or any other appropriate duration. Of course, sampling periods having greater and lesser values than those noted above are contemplated, and the present disclosure is not limited in this regard.

The inventors have contemplated that running out of medicinal fluid to deliver would also produce a protracted drop in pressure on the membrane that could be detected as an upstream occlusion event. In some embodiments of the occlusion detector, the controller estimates the delivered volume of medicinal fluid, compares the delivered volume to the expected total volume, and determines how it should respond to a detected upstream occlusion. Some embodiments rely on the controller estimating the delivered volume. In some embodiments, instead of relying on pre-programmed time based logic, these embodiments of the controller rely on the rotation rate of the motor of the rotary pump to estimate the volume of delivered fluid. Since rotary pumps deliver a known amount of fluid with each rotation, the number of rotations completed by the pump since beginning infusion can provide an estimate for the amount of medicinal fluid delivered. In some embodiments, elapsed time may be included as a factor in estimating delivered volume.

In some embodiments, if the controller determines that under 85% of the expected volume has been delivered, the device alerts the user to any detected upstream occlusions because the infusion process is unlikely to have been completed. If the controller determines that between 85% and 125% of the medicinal fluid is estimated to have been delivered, a detected occlusion, e.g., a third reading, is more likely to be caused by exhaustion of the source medicinal fluid. As such, the device would prompt the user to check if the infusion process is complete. Depending on the embodiment, if the detected occlusion was indeed an occlusion as opposed to the end of delivery, the user can trigger the infusion pump to resume infusing. In some embodiments, the infusion pump is pre-set to deliver another 10% of expected volume, in other embodiments, the infusion pump could deliver another percentage, or could continue with its previous operations. If over 125% of the expected volume of medicinal fluid has been delivered, the infusion pump can prompt the user to confirm if there is still medicinal fluid being delivered.

In the above example, the window of 85%-125% corresponding to the possible end of delivery is based on a 15% tolerance due to flow rate error resulting in faster or slower medicinal fluid delivery. An extra 10% tolerance to the upper end of source volume due to potential over-filling of the medicinal fluid source. However, it should be understood that the percentages provided are working estimates and the current application is not so limited. For example, the lower boundary for the window can be 95% or 90% or 85% or 80% or 75% or 70% or 65% or 60% or 55% or 50% or another percentage or percentage in between one of the aforementioned percentages. The upper boundary for the window can be 100% or 105% or 110% or 115% or 120% or 125% or 130% or 135% or 135% or 140% or 145% or 150% or another percentage or a percentage in between one of the aforementioned percentages. Any combination of the aforementioned upper and lower boundaries are also contemplated. Other combinations are contemplated as well. The controller of an infusion pump of the current disclosure could use any window in practice.

Assuming no issues during the course of infusion, as the medicinal fluid source begins to dwindle, a gradual decline in fluid pressure within the second fluid flow path is expected as the medicinal fluid runs out. In some embodiments, the infusion pump controller monitors for this gradual drop in output signal and alerts a user to the end of delivery when the end of delivery occurs.

In some embodiments, a controller may perform different processes for detecting a downstream occlusion versus an upstream occlusion relative to a pump. It is appreciated that depending on the location of an occlusion and a positon of measurement, the pressure in a fluid line may increase or decrease as a result of an occlusion. For example, when measured at an infusion pump, an occlusion upstream of the infusion pump may cause a decrease is pressure in a fluid line at the infusion pump. In contrast, when measured at an infusion pump, an occlusion downstream of the infusion pump may cause an increase in pressure in the fluid line at an infusion pump. Accordingly, in some embodiments, the controller of an infusion pump may compare a measured pressure or measured average pressure against two different thresholds so that an upstream or a downstream occlusion may be detected. The upstream occlusion pressure threshold is greater than the downstream occlusion pressure threshold, such that the thresholds form a normal operational range of pressures for the infusion pump without occlusions. As described previously, a controller may sample one or more signal outputs (e.g., pressure signal outputs) over a predetermined time period, perform one or operations on the data from said one or more signal outputs, and compare one or more of the signal outputs or a computed variable (e.g., average pressure), to the thresholds. In some embodiments, such a comparison may including increasing or decreasing the value of an incremental variable. This process may be employed for a variety of signal outputs, including force sensor outputs, motor current outputs, and optical sensor outputs, for example.

In some embodiments, it may be desirable to detect an occlusion via one or more sensors that provide different outputs. For example, in some cases, a force sensor used to determine pressure may have too great an uncertainty to determine accurate alarm conditions at certain flow rates of fluid through certain infusion sets. Accordingly, in some embodiments, motor current of an infusion pump may be measured to determine the presence of an occlusion. A controller may sample the motor current (e.g., via an analog-to-digital-converter) over one or more sampling periods during pump operation. In some embodiments, the motor current data sample may be passed through a filter (e.g., a low-pass hardware filter) and/or averaged over a predetermined time period. In some cases, an upstream occlusion may result in an increase in motor current which approximates a step function. Accordingly, a controller may monitor the motor current for the average motor current to exceed a threshold value, whereupon an alarm may be trigged indicating an upstream occlusion condition to a user. Of course, monitoring motor current may be employed for downstream occlusions in some embodiments, as the present disclosure is not so limited. Additionally, both motor current and the signal outputs from one or more pressure or force sensors may be sampled in a particular sampling period, such that there is redundancy in the determination of an alarm condition.

In some embodiments of the bubble detector, the bubble detector includes two pairs of light pipes, spaced from each other, that deliver light through the aforementioned first fluid flow path. The light refracts differently depending on whether air is located within the fluid stream or not. After passing through the first fluid flow path, the light encounters a reflective surface that reflects the light in the opposite direction. Light sensors located below the light pipes detect the reflected light, and send a signal to the controller depending on whether air was detected in the first fluid flow path or not. In some embodiments, the sensor doubles as a light source and the light pipes aid transmission.

Some embodiments of the bubble detector include just a single pair of light pipes, while other light pipes include two, or three, or four, or five, or six, or more pairs of light pipes that may or may not be organized in sub-sets that are spaced from each other. In some embodiments, a single pair of light pipes and a single sensor may be used for the detection of air bubbles. Additional light pipes and light sensors allow the controller to confirm findings, detect the speed of fluid and bubbles, confirm the volume of air bubbles, and perform other functions. In one embodiment of the infusion pump, the controller relies on the output signal generated by multiple light sensors to be within 40% of each other to confirm the existence of a bubble and the associated volume of said bubble. In some embodiments, the bubble detector could also compare accumulation of air independently and process the volume with an average between multiple sensors, or use the maximum or minimum output of one sensor.

In some embodiments, the light pipes are designed to both channel light up to the first fluid flow path, and further allow reflected light to flow down through the light pipe to the sensor. Some embodiments utilize infrared light, but embodiments using other types of light are also contemplated.

In practice, a method of detecting bubbles using the bubble detector is as follows according to some embodiments. The light pipes send light through the first fluid flow path. The light is refracted as it passes through the flow path, establishing a first reading during normal operations or during calibration. The degree of refraction of said light depends on how much air or medicinal fluid is in the flow path, and whether the medicinal fluid is broken up at least partially by air bubbles. The light passes through the flow path and is reflected back by the reflective surface. The light flows through the light pipes to the light sensors. The controller is able to determine whether air was detected in the first fluid flow path or not during at least a second reading, or third reading, etc. In embodiments with redundant light pipes and light sensors, the controller confirms the signal based on readings from the other light sensors. If enough air is detected beyond a threshold, the infusion pump alerts a user to the situation.

According to some embodiments of the infusion pump, there is excess tubing between the outlet of the infusion pump and the needle set fluidly connecting the infusion set hardware and medicinal fluid source to the patient. In some embodiments, this excess tubing could have a volume of 1.5 ml, but other volumes are contemplated. In these embodiments, if the controller has estimated that between 1 ml and 1.5 ml of air has passed the infusion pump, the infusion pump could prompt the user to vent the air. In these embodiments, when prompted, the user can detach the infusion set from the needle set to vent the air before reconnecting the sets and returning to normal infusion operations. It should be understood that the volume of excess tubing is not limited to the provided example of 1.5 ml and could be any volume.

In some embodiments of the infusion pump and bubble detector, the controller monitors the output signal from the light sensors and registers each instance in which a bubble is detected. As air is detected in a sample period, the controller adds 10 $\mu$l to a rolling volume summation. If the summation reaches a threshold volume within a period of time, the controller alerts the user to vent the infusion set. It should be understood that the described values are merely illustrative. Different embodiments of the infusion pump can rely on differing sample periods, differing threshold values, and/or could add differing volumes to the summation during a detected air event. Similarly, according to some embodiments, the infusion pump can utilize a different air bubble processing method. In some embodiments, this threshold volume is 1.5 ml, but other volumes are contemplated as long as the volume can fit the tubing space between the pump engine and the needle set.

The inventors have recognized that the detection of air within the first fluid flow path could correspond to the end of medicinal fluid delivery as opposed to an air bubble. In some embodiments of the infusion pump, the controller raises different alerts depending on how much of the expected medicinal fluid volume has been estimated to have been delivered. These embodiments can estimate volume delivered based on the number of rotations of the rotary pump since the beginning of delivery as earlier described, though other methods of estimation are also contemplated. If the controller determines that under 85% of the expected volume has been delivered, the device alerts the user to vent the excess air that has passed through the pump engine. If the controller determines that between 85% and 125% of the medicinal fluid is estimated to have been delivered, an extended detected bubble could instead indicate that fluid delivery is over. As such, the device would prompt the user to check if the infusion process is complete. Depending on the embodiment, if the infusion process was not complete, the user can trigger the infusion pump to resume infusing. In some embodiments, the infusion pump is pre-set to deliver another 10% of expected volume, in other embodiments, the infusion pump could deliver another percentage, or could continue with its previous operations. If over 125% of the expected volume of medicinal fluid has been delivered, the infusion pump can prompt the user to confirm if there is still medicinal fluid remaining.

The inventors have contemplated that the bubble detector's ability to discern air or fluid in the first fluid flow path could be used to facilitate the priming process for the infusion pump. In some embodiments, when a user is priming the infusion pump before infusion, the bubble detector can detect when the leading edge of the medicinal fluid first reaches the pump engine. When the infusion pump is initially turned on before priming, the bubble detector should detect air in the first fluid flow path. The user can then connect the medicinal fluid source and begin priming the infusion pump. When medicinal fluid arrives at the pump engine, the bubble detector would begin detecting fluid in the fluid flow path. In one embodiment, the infusion pump keeps a 60 second timer beginning when the user begins the priming process. If the bubble detector is still detecting air when the 60 second timer is up, the infusion pump alerts the user to a priming error. When the medicinal fluid has arrived at the pump engine, the user can manually control the pumping and watch the medicinal fluid move from the pump engine to the needle set.

In some embodiments of the infusion pump, the infusion pump controller relies on sensing results from both the occlusion detector and the bubble detector before activating certain alerts. For instance, if a particularly large air bubble is detected, the occlusion detector would also register a drop in pressure that resembles an upstream occlusion. If the controller is presented with a large detected bubble and a detected upstream occlusion, it would alert the user to vent the infusion pump instead of alerting the user to check for an upstream occlusion. It should be understood that this example is illustrative, and the controllers of different embodiments can rely on interplay between the occlusion detector and bubble detector in other ways.

Turning to the figures, specific non-limiting embodiments are described in further detail. It should be understood that the various systems, components, features, and methods described relative to these embodiments may be used either individually and/or in any desired combination as the disclosure is not limited to only the specific embodiments described herein.

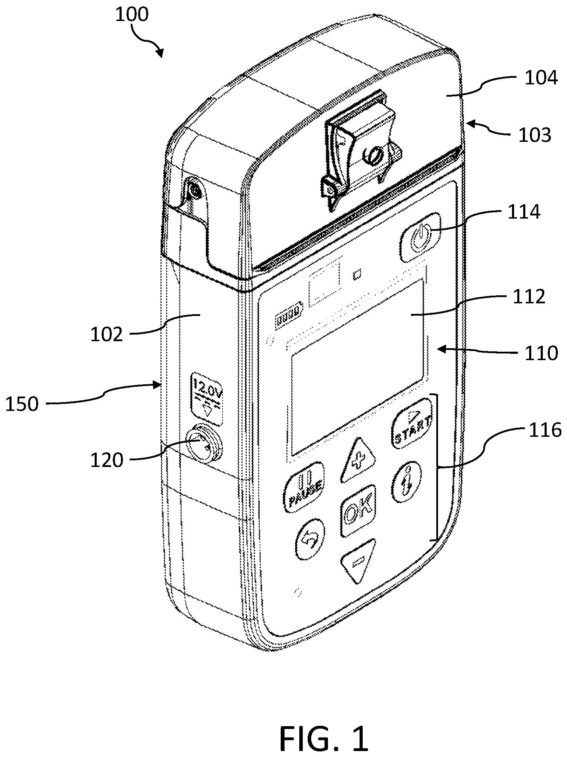

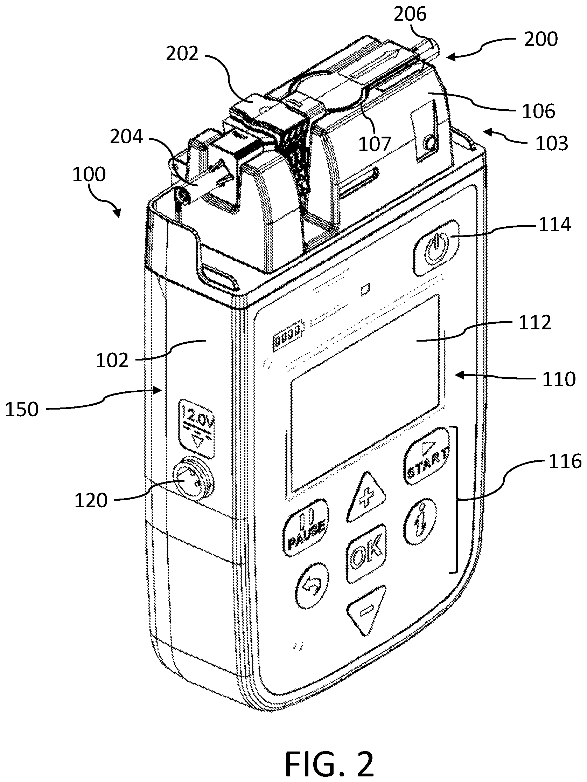

FIG. 1 depicts a perspective view of an embodiment of an infusion pump 100, which is comprised of a pump engine 200, which pumps the fluid from a source to a patient, and a control unit 150 which controls the pump engine. The control unit 150 has a housing 102 and a user interface 110. According to the embodiment of FIG. 1, the housing includes a compartment 103 and contains internal components of the infusion pump 100 such as a motor and controller. The compartment 103 houses pump engine 200 (see FIG. 2). In some embodiments, the pump engine is removably coupled to the compartment. In such embodiments, the compartment 103 may include an openable compartment lid 104 and may be configured to receive a pump engine when opened and secure a pump engine when closed.

The user interface 110 includes a display 112, a power switch 114 configured as a power button, and user controls 116. The display 112 is configured to convey information to an operator of the control unit 150, such as pumping status, instructions for use, alerts, warnings, or any other desirable information. The power button may be used to supply power to a controller and/or motor. As shown in FIG. 1, the control unit 150 includes a power connector 120 for connecting the control unit to a power source configured to supply a source of electrical power. In some embodiments, the control unit may include an internal battery which may be used to selectively supply power to the controller and motor. The user controls 116 may be used to program or select different operational modes of the infusion pump 100, respond to prompts, or otherwise interact with the controller.

FIG. 2 depicts a perspective view of the infusion pump 100 of FIG. 1 with the compartment lid removed. As shown in FIG. 2, the compartment 103 includes a receptacle 106 configured to receive and secure a pump engine 200. In some embodiments, the pump engine is a single-use, disposable component and is thus removably coupled to the compartment. In other embodiments however, the pump engine is a reusable component and may be permanently housed within the compartment 103. The pump engine includes a housing 202, an inlet 204, and an outlet 206. In some embodiments, the pump engine includes a rotor configured to rotate and move fluid from the inlet to the outlet. As discussed previously, the rotor may be configured as any suitable positive displacement rotor or centrifugal pump rotor.

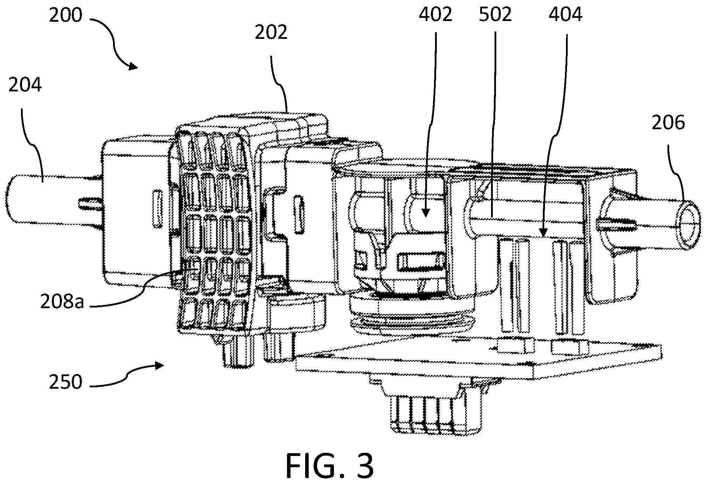

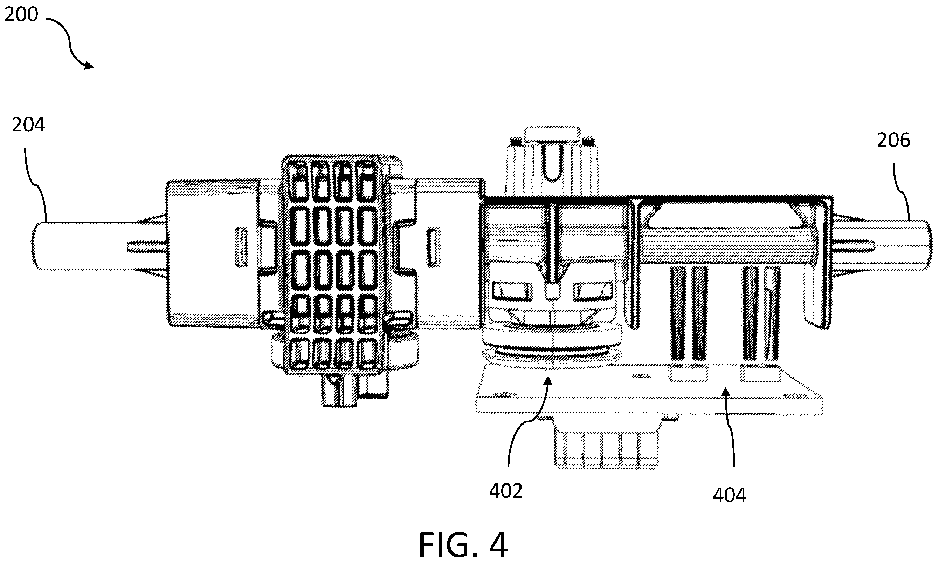

As shown in Figs. 3 and 4, medicinal fluid enters pump engine 200 from a medicinal fluid source via inlet 204, travels along first fluid flow path 502, and exits through outlet 206. Occlusion detector system 402 and bubble detector 404 monitor the medicinal fluid flowing through first fluid flow path 502.

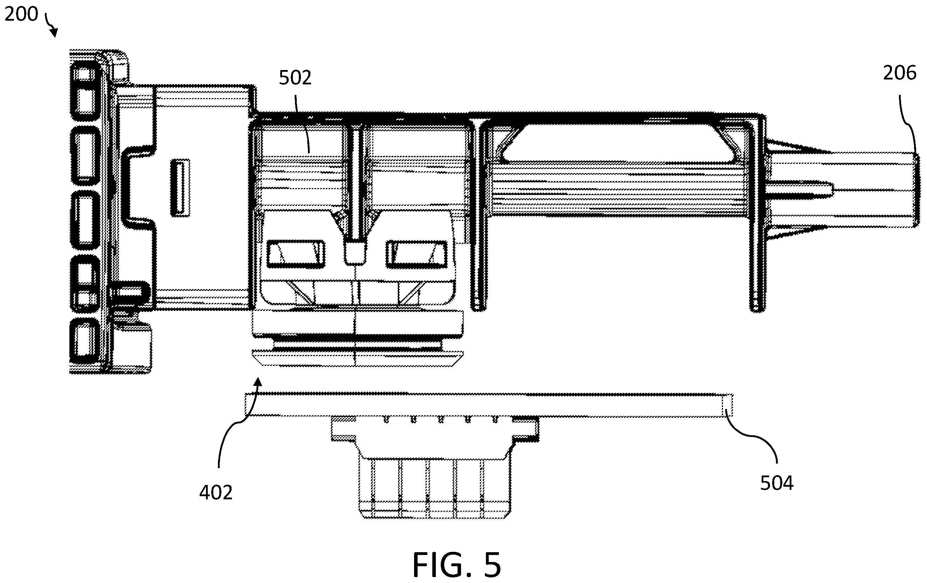

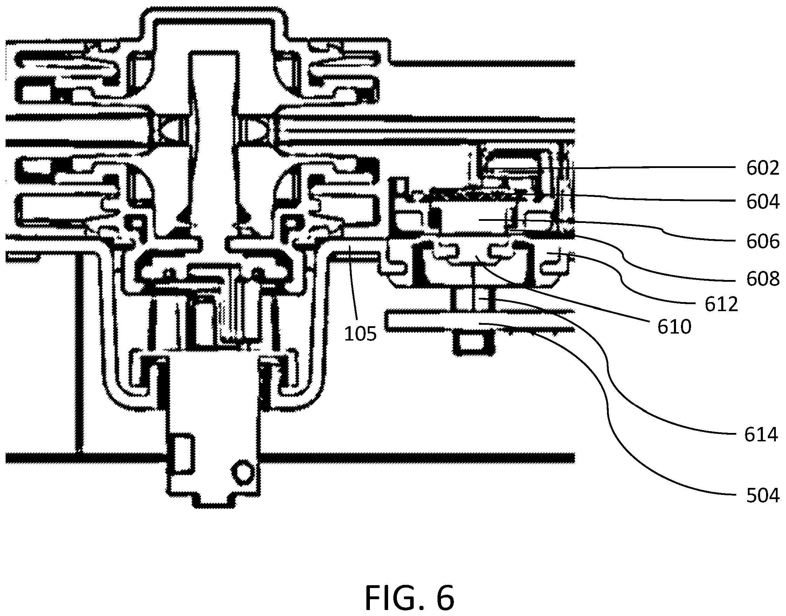

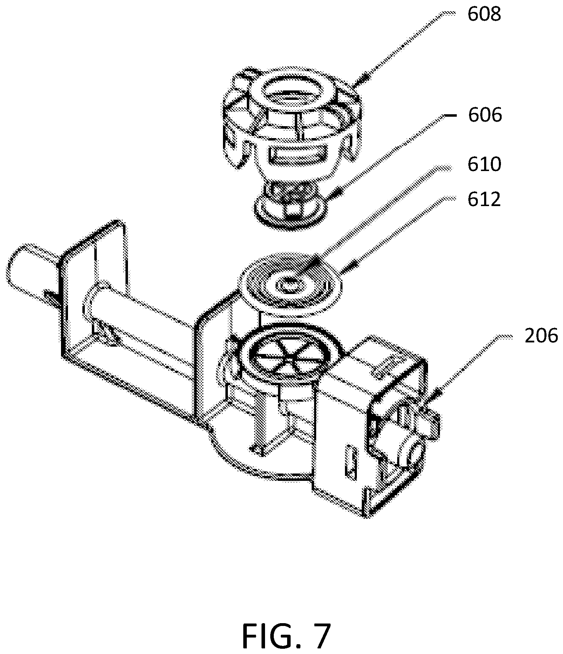

FIG. 5 shows a close-up view of the occlusion detector 402 according to one embodiment with the bubble detector hidden. FIG. 6 shows a partial cross-sectional view of the occlusion detector, and FIG. 7 shows an exploded view of part of the occlusion detector. Second flow path 602 extends substantially perpendicularly from the first fluid flow path, terminating at membrane 604. Retainer 608 forms a fluid-tight seal with membrane 604 to seal off second fluid flow path 602, and further loosely houses piston 606, allowing the piston to freely translate up and down in response to axial force from the membrane 604. When the membrane deforms, the membrane presses force sensor button 610 via the movement of piston 606. In some embodiments, force sensor seal 612 seals against an inner wall 105 to provide a fluid-tight barrier that may help to prevent fluid from the second fluid flow path from leaking down onto the force sensor 614 and the PCB 504 if sealing from the membrane 604 becomes compromised. In some embodiments the force sensor seal retains the force sensor button 610. The force sensor seal may be flexible to permit at least a portion of the seal that is fixed to the force sensor button to be able to move with the force sensor button. In some embodiments, force sensor seal 612 flexes as force is exerted against the force sensor button 610, allowing the force sensor button to move in response to the exertion of force. When the force sensor button 610 is pressed, e.g. due to force being exerted against the button and/or the seal by piston 606, the force sensor button is permitted to move downwardly due to the seal being flexibly deformable, resulting in the force sensor button making contact with the force sensor 614. In the depicted embodiment, the force sensor is a HONEYWELL FSS Low Profile Force Sensor, but other force sensors can be used as described above. The force delivered to the force sensor is translated to an output signal which is relayed to PCB 504, which is part of the controller of the control unit 150. When the external force that was applied to the seal 612 and/or button 610 ceases, the seal 612 automatically returns to its original, pre-deformation configuration, thereby retracting the button 610 away from the force sensor 610 and PCB 504. In some embodiments, the button 610 is embedded within the seal 610. In some embodiments, the button 610 extends completely through the seal such that a portion of the button is exposed on a top side of the seal, and another portion of the button is exposed on the bottom side of the seal. The seal 610 may be formed about the button 610, e.g. by overmolding the seal onto the button. The seal and button may be attached to one another by other techniques, e.g. via bonding, via mechanical fixation, or any other suitable technique.

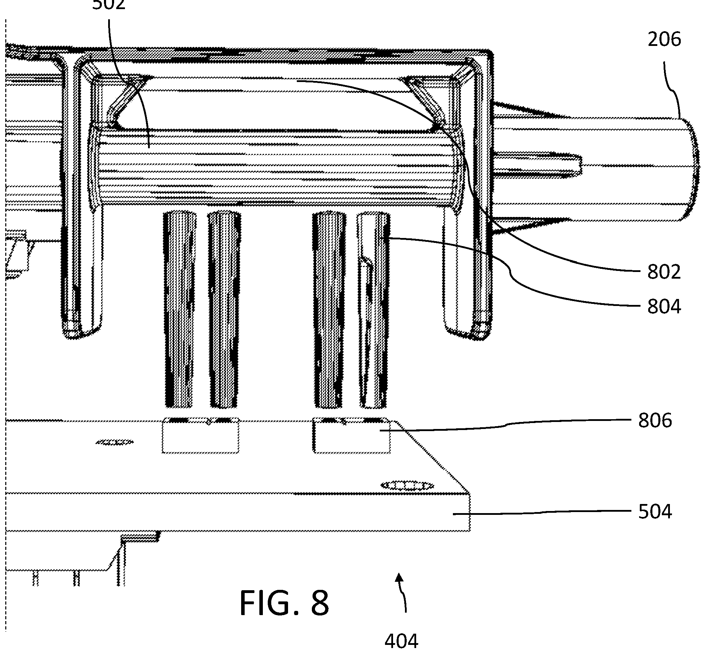

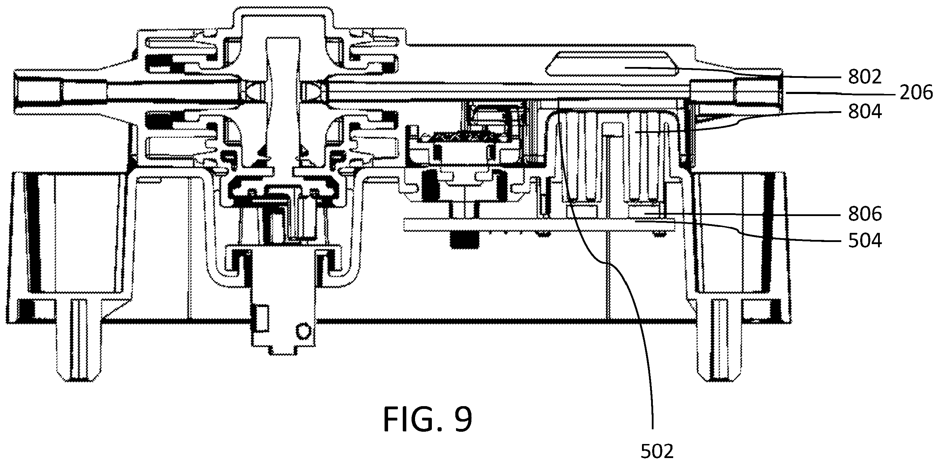

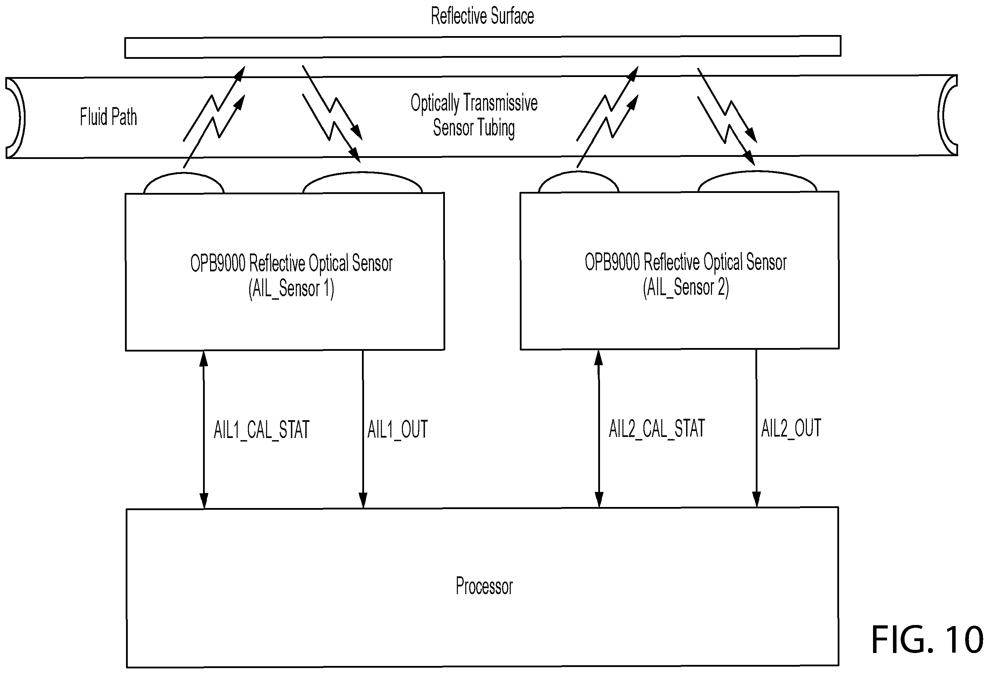

FIG. 8 shows a close-up of the bubble detector according to one embodiment. FIG. 9 shows a partial cross-sectional view of the infusion pump including the bubble detector according to one embodiment. FIG. 10 shows a block diagram illustrating the operation of the bubble detector. Light coming through light pipes 804 is projected through first fluid flow path 502 where the light is refracted differently depending on whether air is present in the flow path or not. The light is then reflected off reflective surface 802 and detected by light sensors 806. In the depicted embodiment, the light sensor 806 is the initial source of the light going through the light pipes. The light sensor in this embodiment is the SMD reflective optical sensor OPB9000 from TT ELECTRONICS. However, other light sensors are also contemplated. Further, embodiments where the light pipes are the source of the light are also contemplated. When the light sensors have processed the reflected light, an output signal is sent to PCB 504.

The above-described embodiments of the technology described herein can be implemented in any of numerous ways. For example, the embodiments may be implemented using hardware, software or a combination thereof. When implemented in software, the software code can be executed on any suitable processor or collection of processors, whether provided in a single computer or distributed among multiple computers. Such processors may be implemented as integrated circuits, with one or more processors in an integrated circuit component, including commercially available integrated circuit components known in the art by names such as CPU chips, GPU chips, microprocessor, microcontroller, or co-processor. Alternatively, a processor may be implemented in custom circuitry, such as an ASIC, or semi-custom circuitry resulting from configuring a programmable logic device. As yet a further alternative, a processor may be a portion of a larger circuit or semiconductor device, whether commercially available, semi-custom or custom. As a specific example, some commercially available microprocessors have multiple cores such that one or a subset of those cores may constitute a processor. Though, a processor may be implemented using circuitry in any suitable format.

Also, the various methods or processes outlined herein may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

The terms "program" or "software" or "firmware" are used herein in a generic sense to refer to any type of computer code or set of computer-executable instructions that can be employed to program a computer or other processor to implement various aspects of the present disclosure as discussed above. Additionally, it should be appreciated that according to one aspect of this embodiment, one or more computer programs that when executed perform methods of the present disclosure need not reside on a single computer or processor, but may be distributed in a modular fashion amongst a number of different computers or processors to implement various aspects of the present disclosure.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Various aspects of the present disclosure may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Also, the embodiments described herein may be embodied as a method, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Further, some actions are described as taken by a "user." It should be appreciated that a "user" need not be a single individual, and that in some embodiments, actions attributable to a "user" may be performed by a team of individuals and/or an individual in combination with computer-assisted tools or other mechanisms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

While the present teachings have been described in conjunction with various embodiments and examples, it is not intended that the present teachings be limited to such embodiments or examples. On the contrary, the present teachings encompass various alternatives, modifications, and equivalents, as will be appreciated by those of skill in the art. Accordingly, the foregoing description and drawings are by way of example only.

EXAMPLE

An example of one way of arriving at the aforementioned thresholds to determine if pressure has reached a level where a downstream occlusion is likely is disclosed below. It should be understood that the following is but one implementation and the current disclosure should not be limited as such.

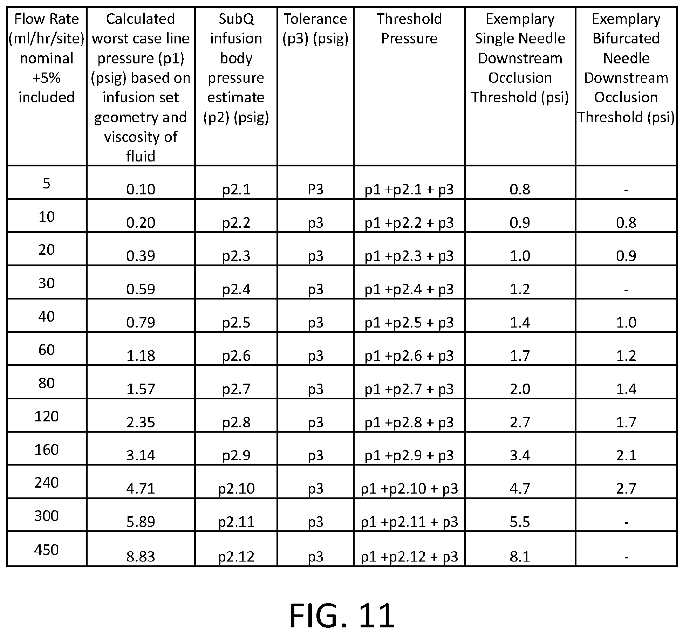

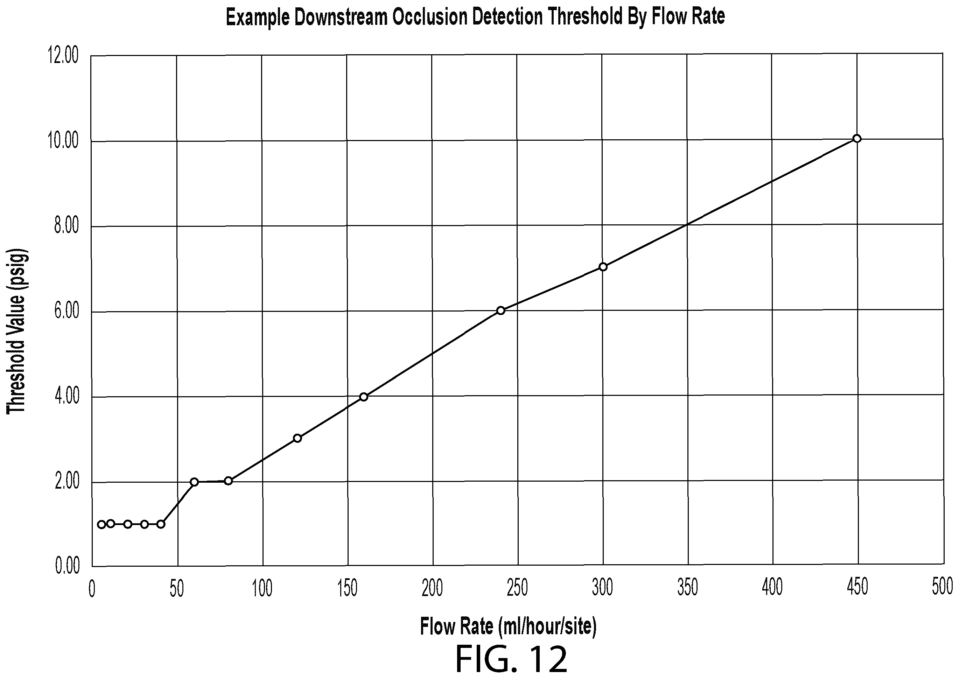

As stated above, one way of arriving at the threshold pressure value for each flow rate is to sum a calculated worst case line pressure (referred to herein as "p1"), with an estimate of body pressure (referred to herein as "p2"), and a tolerance based on possible force sensor error (referred to herein as "p3") to arrive at an acceptable upper limit of fluid pressure. FIGS. 11 and 12 show example values and calculations for the delivery of an example medicinal fluid.

One way to calculate a worst case line pressure is based on the Hagen-Poiseuille equation (1), where P is the pressure difference between the two ends, L is the length of pipe, $\mu$ is the dynamic viscosity, Q is the volumetric flow rate, and d is the pipe diameter.

$$\Delta P = \frac{128\mu LQ}{\pi d^4} \quad (1)$$

Therefore, the difference of pressure in the pipe, or tubing in this case, is dependent upon Flowrate (Q), viscosity of the fluid ($\mu$), and the geometric restriction of the segment based on diameter (d) and length (L). Since the system set is intended to have segments with defined length and diameter, and a constant needle diameter (gauge) and length, the geometric restriction can be planned with knowledge of the tolerances of the segments. Additionally, with therapeutic elements of known viscosity, for each flowrate intended for the system, a value for expected in-line tubing pressure can be calculated.

Turning to body pressure, when delivering fluid to a patient, the backpressure experienced at the needle due to fluid pumped into the body is a variable that must be added to calculate the line pressure. For example, for intravenous delivery, intravenous blood pressure is approximately 2 psi. The backpressure experienced at the needle during subcutaneous delivery may have different values than for intravenous delivery.

It should be mentioned that some embodiments of the infusion pump are designed for use with needle sets that are bifurcated into two needles for infusion into two sites simultaneously. When a needle set having two needles is installed, the pump engine may turn twice as fast as for a needle set having a single needle in order to deliver a programmed flow rate to each of the sites. However, the pressure in line would be approximately the same as a needle set having a single needle at the prescribed flow rate per site, since flow is split between the two needles. In this scenario, if one leg of the bifurcated needle set is restricted, the overall line pressure will increase and can also trigger an occlusion alarm, which could prevent too much fluid from being infused in one site.

## Claims

1. An infusion pump (100), comprising:

   a pump engine (200) having an inlet (204) and an outlet (206), the pump engine being configured to drive fluid in a direction from the inlet to the outlet along a first fluid flow path (502);
   a membrane (604) that is non-porous;
   a piston (606);
   a second fluid flow path (602) in fluid communication with the first fluid flow path, wherein the second fluid flow path terminates at the membrane;
   a force sensor (614); and
   a controller in communication with the force sensor, wherein, in response to deformation of the membrane and piston, the force sensor is configured to send a signal to the controller that reflects fluid pressure experienced in the second fluid flow path,
   wherein:

   a first fluid pressure in the first fluid flow path gives rise to a second fluid pressure in the second fluid flow path,
   the membrane is exposed to and is configured to deform in response to the second fluid pressure in the second fluid flow path, and
   the piston is configured to move and to propagate an axial force to the force sensor in response to deformation of the membrane;

   the infusion pump being **characterized by**:

   a seal (612) that is elastically deformable; and
   a force sensor button (610) attached to the seal, the seal and the force sensor button being positioned between the piston and the force sensor, and being configured such that the seal elastically deforms in response to contact of the piston against the seal or the force sensor button, wherein elastic deformation of the seal causes the force sensor button to move toward the force sensor; wherein the seal forms a fluid-tight barrier that prevents fluid from the second fluid flow path from reaching the force sensor.

2. The infusion pump of claim 1, wherein at least part of the membrane is directly exposed to fluid within the second fluid flow path.

3. The infusion pump of claim 1, wherein the pump engine is a rotary pump.

4. The infusion pump of claim 1, wherein the piston and force sensor are isolated from fluid in the first and second fluid flow paths.

5. The infusion pump of claim 2, wherein the membrane is configured to further deform and increase axial force delivered to the piston when fluid flow is obstructed downstream of the first fluid flow path.

6. The infusion pump of claim 5, wherein the membrane is configured to elastically un-deform and reduce axial force delivered to the piston when fluid flow is obstructed upstream of the first fluid flow path.

7. The infusion pump of claim 1, wherein the second fluid flow path is perpendicular to the first fluid flow path.

8. The infusion pump of claim 1, wherein the pump engine is removable from the infusion pump.

9. A method of monitoring an infusion pump (100), comprising:

determining a threshold value based on a flow rate of a first fluid flow when a pump engine (200) is activated to deliver fluid from an inlet (204) to an outlet (206) across a first fluid flow path (502) such that fluid also enters a second fluid flow path (602) deviating from the first fluid flow path, deforming a non-porous membrane (604) at an end of the second fluid flow path (602) to apply an axial force to a piston (606) operatively connected to a force sensor (614) which delivers a first reading, wherein the piston contacts an elastically deformable seal (612) or a force sensor button (610) attached to the seal to elastically deform the seal and thereby cause the force sensor button to move toward the force sensor, wherein the seal forms a fluid-tight barrier that prevents fluid from the second fluid flow path from reaching the force sensor;
detecting a second reading when an occlusion at least partially obstructs the first fluid flow path, increasing fluid in the second fluid flow path, increasing axial force on the piston through an increase in pressure further deforming the membrane; and
alerting a user if the second reading surpasses the threshold value.

10. The method of claim 9, wherein the second reading surpassing the threshold value further includes the second reading surpassing the threshold value over a plurality of a sampling periods.

11. The method of claim 10, wherein the second reading is an average force over each of the sampling periods.

12. The method of claim 10, further comprising:

detecting a third reading when a reduction of fluid flow in the first fluid flow path decreases fluid in the second fluid flow path, reducing axial force on the piston through a decrease in pressure deforming the membrane; and
alerting the user if the third reading decreases past a low threshold value over the plurality of sampling periods.

13. The method of claim 12, wherein the third reading is an average force over each of the plurality of sampling periods.

**Patentansprüche**

1. Infusionspumpe (100), umfassend:

einen Pumpenmotor (200) mit einem Einlass (204) und einem Auslass (206), wobei der Pumpenmotor so konfiguriert ist, dass er Fluid in einer Richtung vom Einlass zum Auslass entlang eines ersten Fluidfließverlaufs (502) antreibt;
eine Membran (604), die nicht porös ist;
einen Kolben (606);
einen zweiten Fluidfließverlauf (602) in Fluidverbindung mit dem ersten Fluidfließverlauf, wobei der zweite Fluidfließverlauf an der Membran endet;
einen Kraftsensor (614); und
eine Steuerung, die mit dem Kraftsensor in Verbindung steht, wobei der Kraftsensor so konfiguriert ist, dass er als Reaktion auf eine Verformung der Membran und des Kolbens ein Signal an die Steuerung sendet, das den im zweiten Fluidfließverlauf erfahrenen Fluiddruck widerspiegelt,
wobei:

ein erster Fluiddruck im ersten Fluidfließverlauf einen zweiten Fluiddruck im zweiten Fluidfließverlauf hervorruft,
die Membran dem zweiten Fluiddruck im zweiten Fluidfließverlauf ausgesetzt und so konfiguriert ist, dass sie sich als Reaktion darauf verformt, und
der Kolben so konfiguriert ist, dass er sich als Reaktion auf die Verformung der Membran bewegt und eine axiale Kraft auf den Kraftsensor ausbreitet;
wobei die Infusionspumpe **gekennzeichnet ist durch**:

eine Dichtung (612), die elastisch verformbar ist; und
einen Kraftsensorknopf (610), der an der Dichtung befestigt ist, wobei die Dichtung und der Kraftsensorknopf zwischen dem Kolben und dem Kraftsensor positioniert sind und so konfiguriert sind, dass sich die Dichtung als Reaktion auf den Kontakt des Kolbens mit der Dichtung oder dem Kraftsensorknopf elastisch verformt, wobei die elastische Verformung der Dichtung bewirkt, dass sich der Kraftsensorknopf in Richtung des Kraftsensors bewegt;
wobei die Dichtung eine fluiddichte Barriere bildet, die verhindert, dass Fluid aus dem zweiten Fluidfließverlauf den Kraftsensor erreicht.

2. Infusionspumpe nach Anspruch 1, wobei wenigstens ein Teil der Membran dem Fluid innerhalb des zweiten Fluidfließverlaufs direkt ausgesetzt ist.

3. Infusionspumpe nach Anspruch 1, wobei der Pumpenmotor eine Rotationspumpe ist.

4. Infusionspumpe nach Anspruch 1, wobei der Kolben und der Kraftsensor vom Fluid im ersten und zweiten Fluidfließverlauf isoliert sind.

5. Infusionspumpe nach Anspruch 2, wobei die Membran so konfiguriert ist, dass sie sich weiter verformt und die auf den Kolben ausgeübte axiale Kraft erhöht, wenn der Fluidfluss stromabwärts des ersten Fluidfließverlaufs blockiert ist.

6. Infusionspumpe nach Anspruch 5, wobei die Membran so konfiguriert ist, dass sie sich elastisch zurückverformt und die auf den Kolben ausgeübte axiale Kraft reduziert, wenn der Fluidfluss stromaufwärts des ersten Fluidfließverlaufs blockiert ist.

7. Infusionspumpe nach Anspruch 1, wobei der zweite Fluidfließverlauf senkrecht zum ersten Fluidfließverlauf verläuft.

8. Infusionspumpe nach Anspruch 1, wobei der Pumpenmotor aus der Infusionspumpe entfernbar ist.

9. Verfahren zum Überwachen einer Infusionspumpe (100), umfassend:

Bestimmen eines Schwellenwerts auf der Grundlage einer Fließrate eines ersten Fluidflusses, wenn ein Pumpenmotor (200) aktiviert wird, um Fluid von einem Einlass (204) zu einem Auslass (206) über einen ersten Fluidfließverlauf (502) zu fördern, so dass Fluid auch in einen vom ersten Fluidfließverlauf abweichenden zweiten Fluidfließverlauf (602) eintritt, Verformen einer nicht porösen Membran (604) an einem Ende des zweiten Fluidfließverlaufs (602), um eine axiale Kraft auf einen Kolben (606) auszuüben, der betriebsmäßig mit einem Kraftsensor (614) verbunden ist, der einen ersten Messwert liefert, wobei der Kolben eine elastisch verformbare Dichtung (612) oder einen an der Dichtung befestigten Kraftsensorknopf (610) berührt, um die Dichtung elastisch zu verformen und dadurch zu bewirken, dass sich der Kraftsensorknopf in Richtung des Kraftsensors bewegt, wobei die Dichtung eine fluiddichte Barriere bildet, die verhindert, dass Fluid aus dem zweiten Fluidfließverlauf den Kraftsensor erreicht;
Detektieren eines zweiten Messwerts, wenn eine Okklusion den ersten Fluidfließverlauf wenigstens teilweise blockiert, was das Fluid im zweiten Fluidfließverlauf erhöht, die axiale Kraft auf den Kolben durch einen Druckanstieg erhöht, der die Membran weiter verformt; und
Benachrichtigen eines Benutzers, wenn der zweite Messwert den Schwellenwert überschreitet.

10. Verfahren nach Anspruch 9, wobei das Überschreiten des Schwellenwerts durch den zweiten Messwert ferner das Überschreiten des Schwellenwerts durch den zweiten Messwert über eine Vielzahl von Abtastzeiträumen beinhaltet.

11. Verfahren nach Anspruch 10, wobei der zweite Messwert eine durchschnittliche Kraft über jeden der Abtastzeiträume ist.

12. Verfahren nach Anspruch 10, ferner umfassend:

Detektieren eines dritten Messwerts, wenn eine Reduzierung des Fluidflusses im ersten Fluidfließverlauf das Fluid im zweiten Fluidfließverlauf verringert, was die axiale Kraft auf den Kolben durch eine Druckabnahme, die die Membran verformt, reduziert; und
Benachrichtigen des Benutzers, wenn der dritte Messwert über die Vielzahl von Abtastzeiträumen einen niedrigen Schwellenwert unterschreitet.

13. Verfahren nach Anspruch 12, wobei der dritte Messwert eine durchschnittliche Kraft über jeden der Vielzahl von Abtastzeiträumen ist.

**Revendications**

1. Pompe à perfusion (100), comprenant :

   un moteur de pompe (200) ayant une entrée (204) et une sortie (206), le moteur de pompe étant configuré pour entraîner un fluide dans une direction allant de l'entrée à la sortie le long d'un premier chemin d'écoulement de fluide (502) ;
   une membrane (604) qui est non poreuse ;
   un piston (606) ;
   un second chemin d'écoulement de fluide (602) en communication fluidique avec le premier chemin d'écoulement de fluide, dans laquelle le second chemin d'écoulement de fluide se termine au niveau de la membrane ;
   un capteur de force (614) ; et
   un dispositif de commande en communication avec le capteur de force, dans laquelle, en réponse à la déformation de la membrane et du piston, le capteur de force est configuré pour envoyer un signal au dispositif de commande qui reflète la pression de fluide subie dans le second chemin d'écoulement de fluide,
   dans laquelle :

   une première pression de fluide dans le premier chemin d'écoulement de fluide donne lieu à une seconde pression de fluide dans le second chemin d'écoulement de fluide,
   la membrane est exposée et est configurée pour se déformer en réponse à la seconde pression de fluide dans le second chemin d'écoulement de fluide, et
   le piston est configuré pour se déplacer et propager une force axiale au capteur de force en réponse à la déformation de la membrane ;
   la pompe à perfusion étant **caractérisée par** :

   un joint (612) qui est élastiquement déformable ; et
   un bouton de capteur de force (610) fixé au joint, le joint et le bouton de capteur de force étant positionnés entre le piston et le capteur de force, et étant configurés de sorte que le joint se déforme élastiquement en réponse au contact du piston contre le joint ou le bouton de capteur de force, dans lequel la déformation élastique du joint amène le bouton de capteur de force à se déplacer vers le capteur de force ;
   dans laquelle le joint forme une barrière étanche aux fluides qui empêche le fluide du second chemin d'écoulement de fluide d'atteindre le capteur de force.

2. Pompe à perfusion selon la revendication 1, dans laquelle au moins une partie de la membrane est directement exposée au fluide dans le second chemin d'écoulement de fluide.

3. Pompe à perfusion selon la revendication 1, dans laquelle le moteur de pompe est une pompe rotative.

4. Pompe à perfusion selon la revendication 1, dans laquelle le piston et le capteur de force sont isolés du fluide dans les premier et second chemins d'écoulement de fluide.

5. Pompe à perfusion selon la revendication 2, dans laquelle la membrane est configurée pour se déformer davantage et augmenter la force axiale délivrée au piston lorsque l'écoulement de fluide est obstrué en aval du premier chemin d'écoulement de fluide.

6. Pompe à perfusion selon la revendication 5, dans laquelle la membrane est configurée pour se déformer élastiquement et réduire la force axiale délivrée au piston lorsque l'écoulement de fluide est obstrué en amont du premier chemin d'écoulement de fluide.

7. Pompe à perfusion selon la revendication 1, dans laquelle le second chemin d'écoulement de fluide est perpendiculaire au premier chemin d'écoulement de fluide.

8. Pompe à perfusion selon la revendication 1, dans laquelle le moteur de pompe est amovible de la pompe à perfusion.

9. Procédé de surveillance d'une pompe à perfusion (100), comprenant :

   la détermination d'une valeur seuil sur la base d'un débit d'un premier écoulement de fluide lorsqu'un moteur de pompe (200) est activé pour distribuer du fluide d'une entrée (204) à une sortie (206) à travers un premier chemin d'écoulement de fluide (502) de sorte que le fluide pénètre également dans un second chemin d'écoulement de fluide (602) s'écartant du premier chemin d'écoulement de fluide, déformant une membrane non poreuse (604) au niveau d'une extrémité du second chemin d'écoulement de fluide (602) pour appliquer une force axiale à un piston (606) connecté de manière opérationnelle à un capteur de force (614) qui fournit une première lecture, dans lequel le piston entre en contact avec un joint déformable élastiquement (612) ou un bouton de capteur de force (610) fixé au joint pour déformer élastiquement le joint et ainsi amener le bouton de capteur de force à se déplacer vers le capteur de force, dans lequel le joint forme une barrière étanche qui empêche le fluide du second chemin d'écoulement de fluide d'atteindre le capteur de force ;

la détection d'une deuxième lecture lorsqu'une occlusion obstrue au moins partiellement le premier chemin d'écoulement de fluide, augmentant le fluide dans le second chemin d'écoulement de fluide, augmentant la force axiale sur le piston à travers une augmentation de la pression déformant davantage la membrane ; et

l'alerte d'un utilisateur si la deuxième lecture dépasse la valeur seuil.

10. Procédé selon la revendication 9, dans lequel la deuxième lecture dépassant la valeur seuil comporte également la deuxième lecture dépassant la valeur seuil sur une pluralité de périodes d'échantillonnage.

11. Procédé selon la revendication 10, dans lequel la deuxième lecture est une force moyenne sur chacune des périodes d'échantillonnage.

12. Procédé selon la revendication 10, comprenant également :

la détection d'une troisième lecture lorsqu'une réduction d'écoulement de fluide dans le premier chemin d'écoulement de fluide diminue le fluide dans le second chemin d'écoulement de fluide, réduisant la force axiale sur le piston à travers une diminution de la pression déformant la membrane ; et

l'alerte de l'utilisateur si la troisième lecture diminue au-delà d'une valeur seuil basse sur la pluralité de périodes d'échantillonnage.

13. Procédé selon la revendication 12, dans lequel la troisième lecture est une force moyenne sur chacune de la pluralité de périodes d'échantillonnage.

FIG. 1

FIG. 2

FIG. 3

FIG. 4

FIG. 5

EP 3 860 682 B1

FIG. 6

608

606

610

612

206

FIG. 7

FIG. 8

FIG. 9

Reflective Surface

Fluid Path

Optically Transmissive
Sensor Tubing

OPB9000 Reflective Optical Sensor
(AIL_Sensor 1)

OPB9000 Reflective Optical Sensor
(AIL_Sensor 2)

AIL1_CAL_STAT

AIL1_OUT

AIL2_CAL_STAT

AIL2_OUT

Processor

FIG. 10

EP 3 860 682 B1

| Flow Rate (ml/hr/site) nominal +5% included | Calculated worst case line pressure (p1) (psig) based on infusion set geometry and viscosity of fluid | SubQ infusion body pressure estimate (p2) (psig) | Tolerance (p3) (psig) | Threshold Pressure | Exemplary Single Needle Downstream Occlusion Threshold (psi) | Exemplary Bifurcated Needle Downstream Occlusion Threshold (psi) |
|---|---|---|---|---|---|---|
| 5 | 0.10 | p2.1 | P3 | p1 +p2.1 + p3 | 0.8 | - |
| 10 | 0.20 | p2.2 | p3 | p1 +p2.2 + p3 | 0.9 | 0.8 |
| 20 | 0.39 | p2.3 | p3 | p1 +p2.3 + p3 | 1.0 | 0.9 |
| 30 | 0.59 | p2.4 | p3 | p1 +p2.4 + p3 | 1.2 | - |
| 40 | 0.79 | p2.5 | p3 | p1 +p2.5 + p3 | 1.4 | 1.0 |
| 60 | 1.18 | p2.6 | p3 | p1 +p2.6 + p3 | 1.7 | 1.2 |
| 80 | 1.57 | p2.7 | p3 | p1 +p2.7 + p3 | 2.0 | 1.4 |
| 120 | 2.35 | p2.8 | p3 | p1 +p2.8 + p3 | 2.7 | 1.7 |
| 160 | 3.14 | p2.9 | p3 | p1 +p2.9 + p3 | 3.4 | 2.1 |
| 240 | 4.71 | p2.10 | p3 | p1 +p2.10 + p3 | 4.7 | 2.7 |
| 300 | 5.89 | p2.11 | p3 | p1 +p2.11 + p3 | 5.5 | - |
| 450 | 8.83 | p2.12 | p3 | p1 +p2.12 + p3 | 8.1 | - |

# FIG. 11

Example Downstream Occlusion Detection Threshold By Flow Rate

FIG. 12

EP 3 860 682 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5554115 A
- WO 9964091 A2
- US 5661245 A
- US 2003200812 A1